# FASCICULE DE BREVET EUROPEEN

(11) **EP 0 595 683 B1**
(45) Date de publication et mention de la délivrance du brevet: **07.05.1997**
(21) Numéro de dépôt: 93402555.2
(22) Date de dépôt: 18.10.1993
(51) Int. Cl.: A61K 7/00, A61K 7/48, A61K 7/06

(54) **Emulsions eau dans l'huile à phase continue fluorohydrocarbonée et utilisation de certains tensioactifs siliconés pour leur préparation**
Wasser-in-öl Emulsionen mit einer Fluorkohlenwasserstoff enthaltenden kontinuierlichen Phase und die Verwendung von gewissen Silikon Tenziden für ihre Herstellung
Water in oil emulsions with a fluorohydrocarbon containing continuous phase and the use of certain silicone surfactants for their preparation

(30) Priorité: 21.10.1992 FR 9212608
(43) Date de publication de la demande: 04.05.1994
(73) Titulaire: L'OREAL, 75008 Paris (FR)
(72) Inventeur: Mellul, Myriam, F-94240 L'Hay-les-Roses (FR); Arnaud, Pascal, F-94000 Créteil (FR)
(74) Mandataire: Casalonga, Axel

(56) Documents cités:
- EP-A- 0 051 526
- EP-A- 0 158 996
- EP-A- 0 296 661
- EP-A- 0 390 206
- EP-A- 0 422 984
- EP-A- 0 494 412
- WO-A-88/06434
- WO-A-93/11103
- FR-A- 2 450 105
- GB-A- 2 087 882
- US-A- 3 993 744

## Description

La présente invention concerne des émulsions comportant une phase aqueuse dans une phase continue fluorohydrocarbonée, leurs préparations ainsi que leurs utilisations dans les domaines de la cosmétique et de la dermatologie.

Dans le domaine cosmétique notamment, les émulsions eau dans huile E/H sont couramment utilisées car elles permettent de former à la surface de la peau un film qui prévient la perte d'eau transépidermique et protège la peau des agressions extérieures. Compte-tenu des exigences des consommateurs vis-à-vis de ce type d'émulsions qui doivent avoir en même temps une bonne qualité cosmétique qui se traduit en terme d'aspect, de texture et de facilité d'application, de bonnes propriétés protectrices, une bonne conservation de l'efficacité dans le temps (tenue cosmétique) qui supposent de la part du film un pouvoir hydrofuge important, ainsi qu'une bonne résistance à la sueur et au sébum, il est très avantageux de pouvoir obtenir des émulsions présentant toutes ces propriétés sans présenter les inconvénients des émulsions E/H classiques.

En effet, les émulsions E/H, préparées à partir d'huiles hydrocarbonées classiques ou d'huiles siliconées présentent généralement l'inconvénient d'une texture lourde, grasse et/ou collante et une faible tenue cosmétique.

Ainsi, lorsqu'on utilise des huiles de silicone dans la phase continue, si la cosméticité est améliorée, la résistance au sébum reste faible.

Par ailleurs, pour les huiles classiques mentionnées ci-dessus, la substitution d'atomes d'hydrogène par des atomes de fluor entraîne un renforcement des propriétés hydrophobes, filmogènes et lubrifiantes ainsi qu'une certaine lipophobicité. La présence d'atome de fluor était aussi connue pour induire une diminution des propriétés de solubilité dans les matières premières usuelles en cosmétique.

Pour préparer des émulsions E/H, on a donc déjà proposé, pour la phase continue, différents types d'huiles fluorées et notamment les huiles perfluorées où tous les atomes d'hydrogène sont substitués par des atomes de fluor, comme les perfluoropolyéthers et les perfluoroalcanes, les silicones fluorées où une fraction des groupements alkyles portés par le squelette siliconé est fluorée. Cependant aucun de ces types d'huile fluorée n'a pu jusqu'à présent mener à des émulsions convenables.

Ainsi, une première possibilité de formulation des huiles perfluorées dans les systèmes E/H consiste à les disperser, soit dans les émulsions E/H classiques comme le décrivent les brevets EP 196.904 et EP 358.528, soit dans des émulsions siliconées comme le revendique le brevet FR 2 653 125. Cependant leur insolubilité totale (mis à part dans certains solvants fluorés) peut conduire à des distributions non homogènes de ces huiles dans les préparations, entraînant une diminution des performances des produits.

Par ailleurs, leur émulsification sous la forme d'un système E/H perfluorées par des tensioactifs hydrocarbonés conduit le plus souvent à des émulsions très instables, sauf cas particuliers où l'huile fluorée est un chlorofluorocarbure de bas point d'ébullition comme le décrivent les brevets US 3502586 et JP 61-108699 et à condition que la teneur en eau reste faible.

Ces deux derniers brevets ne concernent pas, en outre, le domaine des cosmétiques. Les tensioactifs fluorés permettent par contre d'obtenir des émulsions et des microémulsions du type E/H perfluorées stables comme le décrivent les brevets FR 2184786, EP 250766 et EP 315841 et FR 2630347, mais l'utilisation de ce type de tensioactifs reste limitée en cosmétique.

En ce qui concerne les silicones fluorées, elles ont aussi été proposées pour la préparation d'émulsions E/H siliconées fluorées comme cela est décrit dans les brevets JP 2-298338 et JP 2-295912. Mais, comme dans le cas des huiles perfluorées, la présence de groupements fluorés sur le squelette siliconé induit une diminution importante des propriétés de solubilité avec les composés cosmétiques conventionnels.

Contrairement aux deux classes d'huiles précédentes, les fluorohydrocarbures présentent l'avantage d'être compatibles avec les matières premières couramment utilisées en cosmétique, en particulier les huiles hydrocarbonées et les huiles siliconées.

Compte-tenu de cette compatibilité, il s'est avéré très intéressant de pouvoir émulsifier les fluorohydrocarbures pour obtenir des systèmes E/H qui répondraient à tous les critères évoqués ci-dessus tout en étant faciles à formuler.

La demanderesse a maintenant pu réaliser des émulsions E/H dans lesquelles la phase aqueuse est émulsionnée dans une phase continue constituée d'au moins un fluorohydrocarbure. La phase aqueuse est émulsionnée à l'aide d'un tensioactif siliconé.

Ainsi la présente invention concerne une émulsion du type E/H constituée par une phase aqueuse émulsionnée à l'aide d'un tensioactif siliconé, dans une phase continue comportant un fluorohydrocarbure.

Le terme fluorohydrocarbure désigne des composés dont la structure chimique comporte un squelette carboné dont certains atomes d'hydrogène ont été substitués par des atomes de fluor.

Pour les fluorohydrocarbures, on définit le taux de substitution des atomes d'hydrogène par des atomes de fluor, sous la forme du rapport : nombre d'atomes de fluor/(nombre d'atomes de fluor + nombre d'atomes d'hydrogène) où seuls les atomes d'hydrogène liés aux atomes de carbone du squelette sont pris en compte. Les fluorohydrocarbures ou huiles fluorohydrocarbonées de l'invention comportent au moins un groupement hydrocarboné dans la molécule.

Selon l'invention, en utilisant des tensioactifs siliconés, il est possible d'émulsionner des huiles fluorohydrocarbonées et d'obtenir des émulsions stables avec des huiles fluorohydrocarbonées dont le taux d'atomes de fluor peut être quelconque.

Les fluorohydrocarbures de l'invention ont pour formule la formule I suivante :

(R_{F})ₓ - (A)_{y} - (R_{H})_{z} (I)

dans laquelle :
x représente 1, 2 ou 3,
y représente 0 ou 1,
z représente 0, 1, 2 ou 3,
à la condition que y et z ne soient pas simultanément 0, et que lorsque z est 0, x est 2 ou 3,
R_{F} représente un radical fluoré aliphatique ou aromatique, saturé ou insaturé, à chaîne linéaire, ramifiée ou cyclique, cette chaîne pouvant être fonctionnalisée et/ou être interrompue par des atomes divalents tels que l'oxygène ou le soufre, ou trivalents tels que l'azote et/ou substituée par des atomes d'hydrogène ou d'autres atomes d'halogène, à la condition que, pour deux atomes de carbone du squelette, ne soit pas présent plus d'un de ces substituants autres que le fluor,
R_{H} représente un radical hydrocarboné aliphatique ou aromatique, saturé ou insaturé, à chaîne linéaire, ramifiée ou cyclique, cette chaîne pouvant être fonctionnalisée et/ou interrompue par un ou plusieurs atomes divalents tels que l'oxygène ou le soufre ou par un ou plusieurs atomes trivalents comme l'azote,
A représente un radical di, tri ou quadrivalent tel que 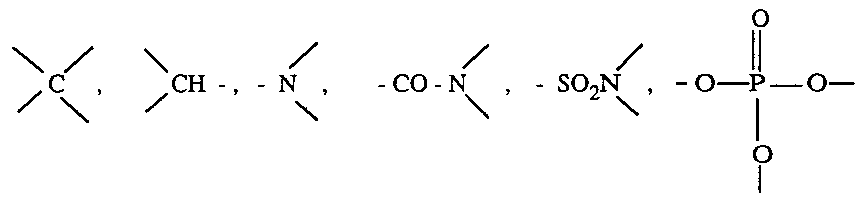 les structures cycliques, aliphatiques ou aromatiques ou les insaturations éthyléniques.

Par fonctionnalisé, on entend selon l'invention, une substitution du squelette, intercalaire, terminale ou pendante, par au moins un groupement organique fonctionnel comme une fonction alcool, thiol, acide, carbonyle, sulfoxyde, ester, amide, amine, phosphate, éthylénique, acéthylénique, et énamine ou sulfonamide.

Par insaturation éthylénique, on entend par exemple 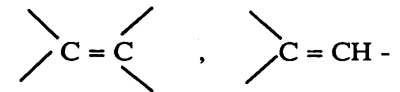 ou -CH=CH-.

De préférence, R_{H} représente un radical alkyle linéaire ou ramifié en C₁-C₂₂ ou un mélange de radicaux alkyle linéaires ou ramifiés en C₁-C₂₂, un radical aryle en C₆-C₁₀ ou un radical aralkyle en C₇-C₁₅.

De préférence, R_{F} représente un radical perfluoroalkyle ayant de 4 à 22 atomes de carbone.

Selon l'invention, les fluorohydrocarbures utilisés ont de préférence un taux de substitution compris entre 0,5 et 95 %. De façon préférée, ce taux est supérieur à 10 % et inférieur à 80 %.

A titre illustratif, on peut citer les composés possédant des groupes perfluorocarbures et des groupes hydrocarbures, le nombre total de carbone étant compris entre 10 et 30, le nombre d'atomes de carbone des groupes hydrocarbures étant égal ou supérieur à deux fois le nombre d'atomes de carbone des groupes perfluorocarbures, tels qu'ils sont décrits dans le document JP 63-002916.

De même, à titre illustratif, on peut citer les fluorohydrocarbures dont la structure générale est définie par la formule (III).

R₁ - (CH₂)ₙ - X -[C₃H₅(OH)]- (Y)ₓ - R₂ (III)

où C₃H₅ (OH) représent : 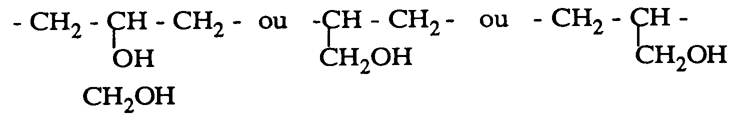
R₁ représente un radical alkyle perfluoré, linéaire ou ramifié en C₄-C₂₀ ou un mélange de radicaux perfluorés, linéaires ou ramifiés en C₄-C₂₀,
R₂ représente un radical alkyle linéaire ou ramifié en C₁-C₂₂ ou un mélange de radicaux alkyle, linéaires ou ramifiés en C₁-C₂₂ ou un radical aryle en C₆-C₁₀ ou aralkyle en C₇-C₁₅,
X et Y, identiques ou différents, représentent : -O-, -S-, 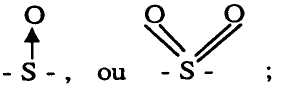 sous réserve que X et Y ne représentent pas simultanément 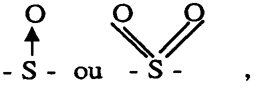
n est compris entre 0 et 4, et
x représente 0 ou 1.

Ces composés utilisés selon l'invention sont décrits dans FR-A-WO 93/11103 et EP-A-166.696.

Par ailleurs, on peut également utiliser, selon l'invention, les composés de formule (IV):

R_{F} - (CH₂)ₙ - X [C₃H₅(OH)] - Y - (CH₂)ₘ - R'_{F} (IV)

dans laquelle C₃H₅(OH) représente les structures : 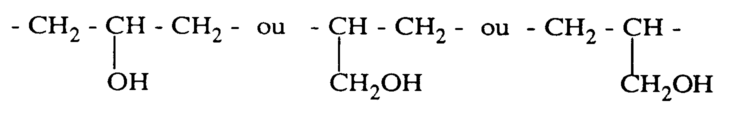
R_{F} et R'_{F}, identiques ou différents, représentent un radical alkyle perfluoré, linéaire ou ramifié en C₄-C₂₀ ou un mélange de radicaux alkyle perfluorés, linéaires ou ramifiés en C₄-C₂₀;
m et n, identiques ou différents, représentent 0, 1, 2, 3 ou 4;
X et Y, identiques, sont - O - ou - S -.

Ces composés sont décrits dans DE-2.702.607, JP 89-193.236, JP 92-275.268 et US-3.893.984.

On peut utiliser aussi les composés de formule :

R_{F} - (CH₂)ₙ - X - [C₃H₅(OH)] - Y - (CH₂)ₘ - R'_{F} (I')

dans laquelle C₃H₅(OH) représente les structures : 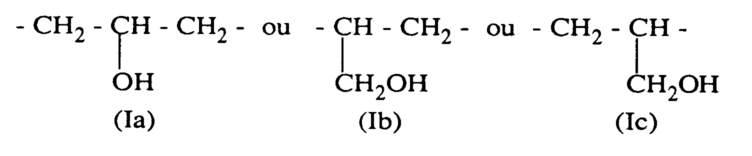
R_{F} et R'_{F}, identiques ou différents, représentent un radical alkyle perfluoré, linéaire ou ramifié en C₄-C₂₀ ou un mélange de radicaux alkyle perfluorés, linéaires ou ramifiés en C₄-C₂₀;
m et n, identiques ou différents, représentent 0, 1, 2, 3 ou 4 et X est O et Y est S ou X est S et Y est O.

Les composés de formule (I') peuvent être préparés en mettant en oeuvre la réaction d'un composé fluoré à hydrogène acide de formule (II'):

R_{F} (CH₂)ₙ- X - H (II')

avec un époxyde de formule (III'): 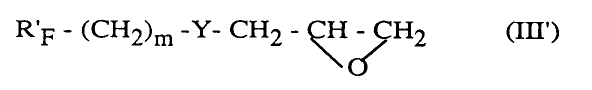 ou la réaction d'un composé fluoré à hydrogène acide de formule (IV'):

R_{F'} - (CH₂)ₘ - Y - H (IV')

avec un époxyde fluoré de formule (V') : 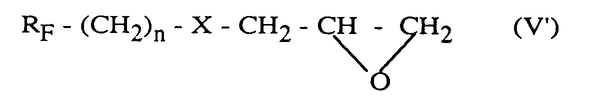 en présence d'un composé basique ou acide jouant le rôle de réactif ou de catalyseur. Les composés sont décrits dans FR 9306605.

On peut aussi utiliser, selon l'invention, les composés décrits dans le document US-3.952.066, de formule: 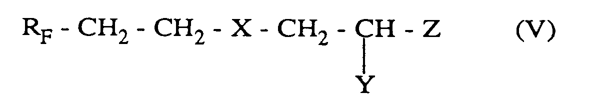 où Y est OH, et
Z est 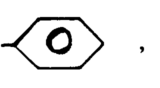 -CH₃, - CH₂OH, CH₂OCOCH₃
ou bien Y est - CH₂OH et Z est O-COCH₃
X représente - O - , - S -, 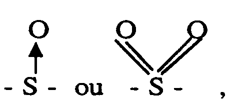 et
R_{F} représente un radical alkyle perfluoré, linéaire ou ramifié, en C₄-C₂₀, ou un mélange de radicaux alkyle perfluorés, linéaires ou ramifiés, en C₄-C₂₀;
ou bien les composés décrits dans le document DE 2.052.579, de formule :

   R_{F} - CH = CH - CH₂ - O - CH₂ - [C₂H₄ - OW] (VI)

   où C₂H₄OW désigne: 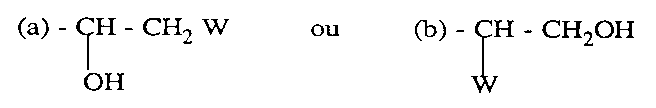
W désignant: -OR, -SR, -COOR, 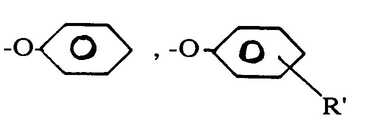
R désigne un radical alkyle en C₁-C₁₈, linéaire ou ramifié,
R' désigne -CH3 ou -OH, en position ortho ou para, et
R_{F} représente un radical alkyle peffluoré, linéaire ou ramifié, en C₄-C₂₀, ou un mélange de radicaux alkyle perfluorés, linéaires ou ramifiés, en C₄-C₂₀.

Par ailleurs, on peut citer, à titre d'exemple, les produits vendus sous la dénomination de NOFABLE FO par la Société NIPPON OIL & Co, ayant la formule suivante : 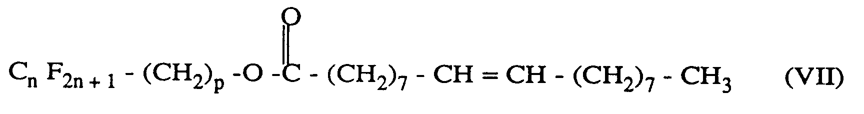 dans laquelle n est un nombre entier égal à 6 ou 8 et p est 1 ou 2.

En plus de l'un ou de plusieurs fluorohydrocarbures, la phase grasse continue peut, selon l'invention, contenir d'autres composés usuellement utilisés dans le domaine cosmétique. Il peut s'agir de composés actifs mais aussi de composés usuellement utilisés à titre d'excipient.

L'ensemble de ces additifs, selon l'invention, n'excède de préférence pas 50 % en poids par rapport au poids de la phase continue grasse.

On peut citer comme additif, les huiles et cires hydrocarbonées suivantes :
- les huiles minérales telles que l'huile de paraffine, l'huile de vaseline et les huiles minérales ayant un point d'ébullition compris entre 310 et 410°C,
- les huiles d'origine animale telles que le perhydrosqualène,
- les huiles végétales telles que l'huile d'amande douce, l'huile de sésame, l'huile de calophyllum, l'huile de palme, l'huile d'avocat, l'huile de jojoba, l'huile d'olive, l'huile de ricin, les huiles de germes de céréales telles que l'huile de germes de blé,
- les esters de synthèse tels que l'huile de Purcelin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, le dicaprylate de propylène glycol, l'adipate de diisopropyle,
- les alcools organiques tels que l'alcool oléique, l'alcool linoléique, l'alcool linolénique, l'alcool isostéarylique, l'octyl dodécanol,
- les esters dérivés d'acide lanolique tels que le lanolate d'isopropyle, le lanolate d'isocétyle,
- les acétylglycérides, les octanoates et décanoates d'alcools et de polyalcools tels que ceux de glycol et de glycérol, les ricinoléates d'alcools et de polyalcools tels que celui de cétyle,
- les cires minérales telles que les cires microcristallines, la paraffine, la vaseline, la cérésine,
- les cires fossiles telles que l'ozokérite, la cire de montan,
- les cires d'origine animale telles que la cire d'abeilles, le spermaceti, la cire de lanoline, les dérivés issus de la lanoline tels que les alcools de lanoline, la lanoline hydrogénée, la lanoline hydroxylée, la lanoline acétylée, les acides gras de la lanoline, l'alcool de lanoline acétylée,
- les cires d'origine végétale telles que la cire de candellila, la cire de Carnauba, la cire du Japon, le beurre de cacao,
- les cires synthétiques comme les cires de polyethylène,
- les huiles hydrogénées concrètes à 25°C telles que l'huile de ricin hydrogénée, l'huile de palme hydrogénée, le suif hydrogéné, l'huile de coco hydrogénée,
- les esters gras concrets à 25°C tels que le monomyristate de propylène glycol, le myristate de myristyle,
- parmi les cires, on peut également citer : l'alcool cétylique, l'alcool stéarylique, les mono-, di- et triglycérides concrets à 25°C, le monoéthanolamide stéarique, la colophane et ses dérivés tels que les abiétates de glycol et de glycérol, les sucro-glycérides et les oléates, myristates, lanolates, stéarates et dihydroxystéarates de calcium, magnésium, zinc et aluminium.

On peut également citer les composés siliconés suivants : les diméthylpolysiloxanes cycliques, les diméthylpolysiloxanes de faible et/ou de haute viscosité, les gommes de silicone, des organopolysiloxanes comme les phénylméthylpolysiloxanes et les phényltriméthylsiloxypolysiloxanes, les alkylméthylpolysiloxanes, les alcoxyméthylpolysiloxanes, des silicones comportant des groupements fonctionnels comme les fonctions alcool ou amine ou thiol.

Par ailleurs on peut également citer les huiles perfluorées suivantes :
les huiles appartenant au groupe des perfluoroalcanes, des perfluorocycloalcanes, des perfluoropolycycloalcanes et des perfluoro (alkylcycloalcanes), mais aussi celles appartenant au groupe des hydrocarbures perfluorés aromatiques ou encore ceux appartenant au groupe des hydrocarbures perfluorés contenant au moins un hetéroatome comme les amines tertiaires, les composés hétérocycliques saturés ou enfin les perfluoropolyéthers.

On peut également incorporer à la phase grasse continue les gélifiants huileux comme par exemple :
- les esters métalliques tels que le stéarate de polyoxyaluminium, ou l'hydroxystéarate d'aluminium et de magnésium,
- les esters d'acides gras et de glycol et les triglycérides,
- les mélanges d'alcools gras,
- les dérivés de cholestérol notamment l'hydroxycholestérol,
- des minéraux argileux gonflants avec les huiles appartenant au groupe des montmorillonites.

La phase grasse continue peut en outre contenir des filtres, des vitamines, des hormones, des antioxydants, des conservateurs, des colorants, des parfums et tout additif lipophile habituellement utilisé en cosmétique.

En ce qui concerne la phase aqueuse de l'émulsion selon l'invention, celle-ci représente 10 à 90 % en poids de la formule totale de l'émulsion.

La phase aqueuse peut contenir outre l'eau, un certain nombre d'autres constituants hydrosolubles souvent utilisés dans le domaine cosmétique, tels que :
. des polyols comme le propylèneglycol, le butylène 1,3-glycol, le glycérol et le polyglycérol, le sorbitol, le glucose ou encore la saccharose, dans des proportions ne dépassant pas 80% en poids par rapport à la phase aqueuse.
. des gélifiants aqueux comme :
   - les polysaccharides tels que les dérivés cellulosiques (carboxy méthylcellulose, hydroxypropylméthylcellulose, ...) et aussi la gomme de xanthane ou de caroube.
   - les protéines tels que la kératine sulfonique, le collagène ou l'élastine.
   - les silicates tels que le silicate d'aluminium et de magnésium.
   - les dérivés acryliques tels que les carbomers et le polyacrylate de glycérol.
   - les polyéthylèneglycols.
. des actifs tels que le hyaluronate de sodium, le sel de sodium de l'acide pyroglutamique, le gluconate de magnésium, des oligo-éléments et des dérivés biologiques, le polyméthacrylate de glycéryle.
. des sels tels que le sulfate de magnésium ou le chlorure de sodium.
. des minéraux argileux gonflant en milieu aqueux comme la saponite, l'hectorite ou encore la smectite.
. des acides aminés.
. des colorants.

Selon l'invention, la phase aqueuse est émulsionnée avec un tensio-actif siliconé de formule générale (II) suivante : 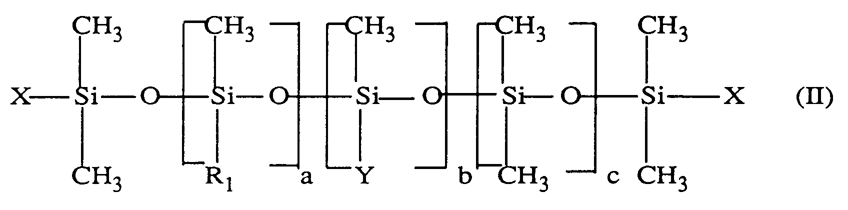 dans laquelle :
X représente CH₃, (CH₂)ₚ-(C₂H₄O)ₘ-(C₃H₆O)ₙ-R₂
Y représente (CH₂)ₚ-O-(C₂H₄O)ₘ-(C₃H₆O)ₙ-R₂
R₁ représente un radical alkyle en C₂-C₁₈
R₂ représente H, un radical alkyle en C₁-C₅, un radical aryle ou un radical acétyle,
p = 1 à 5
m = 0 à 50
n = 0 à 30, m et n n'étant pas simultanément 0
a = 0 à 100
b = 1 à 50
c = 0 à 300.

De préférence, dans cette formule (II), a et c ne sont pas simultanément nuls.

De tels tensioactifs sont des produits commerciaux et on peut citer à titre d'exemple, parmi les alkyldiméthicones copolyols, c'est-à-dire les composés où a n'est pas nul, les composés vendus sous les dénominations :
- "ABIL WS 08", "ABIL WE 09", "ABIL EM 90" par la Société GOLDSCHMIDT,
- "Q2 5200" par la Société DOW CORNING, et
- "218-1138" par la Société GENERAL ELECTRIC.

Parmi les diméthicones copolyols, c'est-à-dire les composés pour lesquels a est nul, on peut citer notamment les composés vendus sous les dénominations :
- "KF 6015", "KF 6017", "X 224013" par la Société SHIN ETSU CHEMICAL,
- "SLM 55033" par la Société WACKER, et
- "Q2 - 3225 C" par la Société DOW CORNING.

A titre d'exemple, également, on peut citer les tensio-actifs siliconés de formule (VIII) suivante : 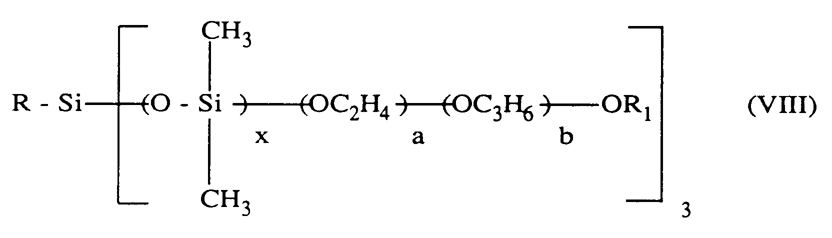
où R et R₁ sont des groupes alkyle en C₁ à C₈,
a et b ont une valeur comprise entre 0 et 200,
a et b n'étant pas simultanément nuls et
x a une valeur comprise entre 1 et 100.

De préférence, a et b ont une valeur comprise entre 0 et 50.

Parmi les composés de ce type, on peut citer les composés commercialisés par la Société UNION CARBIDE sous les dénominations "SILWET L-720" et "SILWET L-722".

Selon l'invention, ces tensioactifs siliconés sont utilisés dans des proportions comprises entre 0, 5 et 40 % en poids par rapport au poids de l'émulsion. De préférence, ces proportions sont comprises entre 2 et 10 % en poids.

Lorsque l'on utilise les dimethicones copolyols, on les utilise de préférence en association avec au moins un tensioactif hydrocarboné dont le HLB (hydrophilic lipophilic balance) est inférieur à 7. Dans ce cas, le tensioactif hydrocarboné est employé dans des proportions variant de 0 à 5 % en poids par rapport au poids total de l'émulsion.

A titre d'exemple, parmi les tensioactifs hydrocarbonés usuels, on peut citer les sels métalliques d'acides gras tel que le lanolate de magnésium, les esters d'acide gras et du glycérol, du polyglycérol ou encore du sorbitol tels que le monoglycéride d'acide oléique, le diisostéarate de polyglycéryle ou le monoisostéarate de sorbitan, et les esters de sucrose tel que le distéarate de saccharose.

Par ailleurs, les émulsions de l'invention peuvent contenir des produits pulvérulents, dans des proportions comprises entre 0 et 95 % en poids par rapport au poids de l'émulsion. A titre d'exemple, parmi les produits pulvérulents d'origine naturelle ou synthétique, on peut citer des poudres végétales telles que l'amidon de maïs, de froment ou de riz, des poudres minérales telles que le talc, le kaolin, le mica, la silice, les silicates, l'alumine, les zéolites, l'hydroxyapatite, la séricite, le dioxyde de titane, les micatitanes, l'oxyde de zinc, le sulfate de baryum, les oxydes de fer, le violet de manganèse, l'oxyde de chrome, le bleu d'outremer et l'oxychlorure de bismuth ou encore le nitrure de bore, des poudres métalliques telles que la poudre d'aluminium, des poudres organiques telles que les poudres de nylon, les poudres de polyamide, les poudres de polyester, les poudres de cellulose, les poudres de polyéthylène, les poudres de polypropylène, les poudres de polystyrène, et les poudres de polytétrafluoroéthylène, et des pigments organométalliques associant le zirconium, le baryum ou l'aluminium à des colorants organiques.

Ces produits pulvérulents cités peuvent être éventuellement enrobés par des sels métalliques d'acides gras, des acides aminés, de la lécithine, du collagène, du polyéthylène, des composés siliconés, des composés fluorés, des composés fluorosiliconés ou tout autre enrobage usuel.

Pour préparer les émulsions de l'invention, on suit un mode opératoire usuel et similaire à celui des émulsions E/H classiques ou siliconées.

Un autre objet de l'invention est constitué par l'utilisation des tensio-actifs siliconés de formule (II) ou (VIII) pour la préparation des émulsions selon l'invention.

Un autre objet de l'invention est constitué par un procédé de préparation d'une émulsion du type E/H comportant une phase aqueuse émulsionnée par un tensioactif siliconé, dans une phase grasse continue comportant au moins un fluorohydrocarbure, caractérisé en ce que le tensioactif est solubilisé ou dispersé dans la phase grasse, et la phase aqueuse est incorporée au mélange sous agitation.

Les fluorohydrocarbures de l'invention peuvent se présenter, à l'état pur, sous différents aspects physiques, c'est-à-dire qu'ils peuvent être liquides, pâteux ou encore solides. Pour préparer l'émulsion, les différents constituants de la phase grasse sont pesés ensemble ou séparément. Ils sont alors portés à une température où ils se trouvent tous à l'état liquide ou solubilisé et alors mélangés.

Par ailleurs, le ou les tensioactifs sont alors solubilisés ou dispersés à chaud dans la phase grasse.

Lorsque des produits pulvérulents sont prévus dans la phase grasse, ils sont alors incorporés dans celle-ci.

La phase aqueuse est incorporée, sous agitation vigoureuse à la phase grasse et l'émulsion peut alors être ensuite refroidie jusqu'à la température ambiante, de préférence progressivement et en conservant l'agitation.

Les émulsions obtenues, à température ambiante, peuvent se présenter sous différents aspects physiques. L'aspect physique des émulsions obtenues est lié à la nature des constituants présents dans chacune des phases, phase aqueuse et phase continue grasse ainsi qu'à la proportion respective d'une phase par rapport à l'autre.

Les émulsions ainsi obtenues sont stables pendant plusieurs mois dans une large gamme de températures comprises entre -4 et + 45°C et résistent à l'épreuve de centrifugation de 4.000 tours/mn pendant une heure.

Par ailleurs, en plus du fait signalé ci-dessus, que des émulsions peuvent être préparées pour une très large gamme de la valeur du taux de substitution, il est apparu que des émulsions selon l'invention peuvent être préparées pour de larges gammes de teneur en eau de l'émulsion.

Ainsi, de manière similaire aux émulsions où la phase continue est hydrocarbonée ou siliconée, il est possible d'obtenir des émulsions de viscosité très différentes allant du très fluide à l'état solide en jouant sur le pourcentage de la phase aqueuse et/ou en choisissant des constituants gélifiant ou structurant dans chacune des phases.

Pour la réalisation de l'émulsion du type E/H, la phase continue comportant le fluorohydrocarbure ne doit pas être miscible avec la phase aqueuse. Ainsi, le fluorohydrocarbure ne doit pas être hydrosoluble.

De même, l'ajustement des indices de réfraction entre la phase grasse et la phase aqueuse par addition de polyols dans cette dernière permet d'obtenir des émulsions transparentes et ceci avec un pourcentage de polyols d'autant plus bas que le taux de fluor dans la phase grasse est élevé.

Ces émulsions comportent par ailleurs de bonnes propriétés sensorielles liées à la présence des fluorohydrocarbures : elles sont en effet confortables, très faciles à appliquer et conduisent à la formation d'un film de très faible épaisseur, très doux, très uniforme, répondant aux exigences de protection et de tenue.

Compte tenu de ces propriétés et de la large gamme d'émulsions que l'on peut obtenir selon l'invention, les applications des émulsions selon l'invention dans le domaine cosmétique et dermatologique sont multiples. Ces applications concernent aussi bien des produits blancs que des produits colorés. Ces émulsions selon l'invention peuvent se présenter sous la forme de lait, de crème de soin pour la peau ou les cheveux, de crème anti-solaire, de crème teintée, de fond de teint, de rouge à lèvres, de mascara ou de fard à joues.

Les émulsions de l'invention ou les compositions cosmétiques préparées avec ces émulsions peuvent donc être utilisées pour le traitement ou le soin de la peau, des cheveux ou des ongles.

D'autres caractéristiques et avantages de l'invention apparaîtront à la lecture des exemples ci-après et qui sont destinés à l'illustrer sans en limiter la portée.

### Exemples de préparation de tensio-actifs fluorohydrocarbonés.

### EXEMPLE I

### 1-(2'-F-hexyléthylthio) 3-(2''-éthylhexyloxy)-2-propanol

### En présence de méthylate de sodium :

A la température de 25°C, sous agitation et sous courant d'azote, on ajoute à 152 g de 2-F-hexyléthanethiol, 3,6 g d'une solution méthanolique de méthylate de sodium (environ 30% - 5,54 meq g⁻¹) en une minute.

Le mélange est chauffé à 70°C. On évapore sous vide le méthanol présent dans le milieu.

Le 2-éthylhexylglycidyléther (74,4 g) est ensuite ajouté goutte-à-goutte en une heure. On maintient la température du mélange entre 60 et 70°C au cours de l'addition de l'époxyde.

A la fin de l'addition, la température est amenée à 25°C.

Le mélange est neutralisé à l'aide de 20 ml de HCl Normal.

Le 1-(2'-F-hexyléthylthio)3-(2''-éthylhexyloxy)2-propanol est séparé par distillation : Eb = 141°C/66,5 Pa.

On obtient 175 g (77%) d'une huile translucide incolore.

| Analyse élémentaire : | | | | |
|---|---|---|---|---|
| | % C | % H | % S | % F |
| Calculé | 40,28 | 4,80 | 5,66 | 43,60 |
| Mesuré | 40,37 | 4,82 | 5,55 | 43,74 |

### EXEMPLE II

### 1-(2'-F-octyléthylthio)3-butyloxy 2-propanol

Selon le mode opératoire décrit à l'exemple 1, on condense en 1 heure 40 g (0,31 mole) d'éther de butyle et de glycidyle sur 147,7 g (0,31 mole) de 2-F-octyléthanethiol, en présence de 2,75 g de solution méthanolique de méthylate de sodium (5,54 meq g⁻¹). En fin de réaction, le mélange est neutralisé par 15,5 ml de HCl Normal.

Après distillation (138° - 142°C/6,65 Pa), on obtient 153 g de 1-(2'-F-octyléthylthio)3-butyloxy 2-propanol sous forme d'une huile incolore.

Rendement = 80%.

| Analyse élémentaire : | | | | |
|---|---|---|---|---|
| | % C | % H | % S | % F |
| Calculé | 33,45 | 3,14 | 5,25 | 52,92 |
| Mesuré | 33,52 | 3,23 | 5,14 | 52,67 |

### EXEMPLE III

### 1-(2'-F-hexyl-éthylthio) 3-(2'-F-hexyl-éthoxy)-2-propanol

A la température de 25°C, sous agitation et sous courant d'azote, on ajoute 1,33 g d'une solution méthanolique de méthylate de sodium (environ 30% - 5,65 meq g⁻¹) à 57 g de 2-F-hexyl-éthanethiol, en une minute.

Le mélange est chauffé à 70°C. On évapore sous vide le méthanol présent dans le milieu.

Le 2-F-hexyl-éthylglycidyléther (63 g-0,15 mole) est ensuite ajouté goutte-à-goutte en une heure. On maintient la température du mélange entre 60 et 70°C au cours de l'addition de l'époxyde.

A la fin de l'addition, la température est amenée à 25°C

Le mélange est neutralisé à l'aide de 7,5 ml de HCl Normal.

Le 1-(2'-F-hexyl-éthylthio)3-(2'-F-hexyl-éthoxy)-2-propanol est séparé par distillation : Eb = 170°C/133 Pa.

On obtient 85 g (71%) d'une huile translucide incolore.

| Analyse élémentaire : | | | | |
|---|---|---|---|---|
| | % C | % H | % S | % F |
| Calculé | 28,51 | 1,76 | 4,01 | 61,72 |
| Mesuré | 28,60 | 1,79 | 4,32 | 61,54 |

### EXEMPLE IV

### 1-(2'-F-hexyl-éthylthio) 3-octylthio-2-propanol

A la température de 25°C, sous courant d'azote, on ajoute 0,61 g d'une solution méthanolique de méthylate de sodium (environ 30% - 5,65 meq g⁻¹) à 10,05 g (0,069 mole) d'octanethiol.

Le mélange est chauffé à 70°C. On évapore sous vide le méthanol présent dans le milieu.

Le 2-F-hexyl-éthylthioglycidyléther (30 g-0,069 mole) est ensuite ajouté goutte-à-goutte en 30 minutes. On maintient la température du mélange entre 60 et 70°C au cours de l'addition de l'époxyde.

A la fin de l'addition, la température est amenée à 25°C.

Le mélange est neutralisé à l'aide de 3,5 ml de HCl Normal.

Le 1-(2'-F-hexyl-éthylthio)3-octylthio-2-propanol est séparé par distillation : Eb = 178°C/66,5 Pa.

On obtient 30 g (75%) d'une huile transparente incolore.

| Analyse élémentaire : | | | | |
|---|---|---|---|---|
| | % C | % H | % S | % F |
| Calculé | 39,18 | 4,67 | 11,01 | 42,40 |
| Mesuré | 39,16 | 4,65 | 10,57 | 42,46 |

### Exemples de préparation d'émulsions.

### Exemples 1 à 9 (2, 4, 6, 8 comparatifs)

Des émulsions sont obtenues de la manière suivante :

On solubilise ou on disperse 10, 0 g de tensioactif dans 20,0 g de fluorohydrocarbure à une température de 70°C. Puis, on ajoute lentement sous agitation 70, 0 g d'eau en conservant au cours de l'addition, une température minimale de 50°C.

On laisse ensuite revenir progressivement à température ambiante en maintenant l'agitation.

On utilise les tensioactifs suivants :
- **Tensioactif hydrocarboné (TAH)** (exemples 4, 8) Isostearyl diglyceryl succinate vendu sous la dénomination "IMWITOR 780 K" par la Société HULS FRANCE
- **Tensioactif siliconé (TAS)** (exemples 1, 3, 5, 7, 9) Cetyldimethicone copolyol vendu sous la dénomination d'"ABIL EM 90" par la Société GOLDSCHMIDT
- **Tensioactif fluoré (TAF)** (exemples 2, 6)
   C₆F₁₃-C₂H₄-S-C₂H₄O(C₂H₄O)₂-H décrit dans FR 2 565226.
On utilise les fluorohydrocarbures suivants :
A : C₆F₁₃-CH=CH-C₁₆H₃₃ (exemples 1, 2)
B : 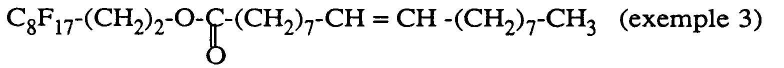
C : 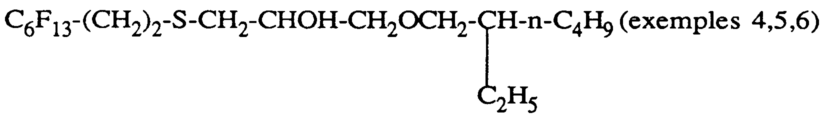
D : C₈F₁₇-(CH₂)₂-S-CH₂-CHOH-CH₂O-n-C₄H₉ (exemple 7)
E: C₆F₁₃-CH=CH₂ (exemples 8, 9)
F: C₆F₁₃-(CH₂)₂-S-CH₂-CHOH-CH₂-O(CH₂)₂C₆F₁₃
G: C₆F₁₃-(CH₂)₂-S-CH₂-CHOH-CH₂-S-C₈H₁₇

I :

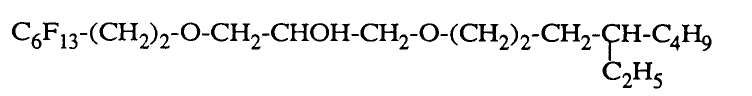

Les émulsions obtenues sont observées et les résultats apparaissent au tableau I ci-après.

**TABLEAU I**

| FLUOROHYDROCARBURE | % F | EXEMPLES | | |
|---|---|---|---|---|
| | | TAH | TAS | TAF |
| A | 27,1 | | 1 +++ | 2 -- |
| B | 31,5 | | 3 +++ | |
| C | 33,3 | 4 - | 5 +++ | 6 -- |
| D | 48,5 | | 7 ++ | |
| E | 81,3 | 8 - | 9 + | |
| F | 66,7 | | ++ | -- |
| G | 33,3 | | +++ | |
| I | 33,3 | | +++ | |

| | | | | |
|---|---|---|---|---|
| + + + *Emulsion très fine à fine, stable* | | | | |
| + + *Emulsion moyenne, stable* | | | | |
| + *Emulsion assez grossière, stable* | | | | |
| - *Emulsion grossière, instable* | | | | |
| -- *Emulsion très instable, décantation rapide en deux phases.* | | | | |

### Exemples 10 à 15 (4, 6, 10, 12, 13, 15 comparatifs)

Les émulsions sont préparées selon le mode opératoire décrit pour les exemples 1 à 9, à partir du 1-(2'-F-hexyléthylthio)-3 -(2''-éthylhexyloxy)-2-propanol, fluorohydrocarbure C de formule :

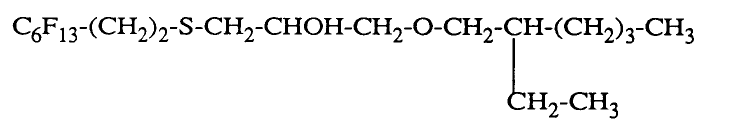

On utilise les tensioactifs utilisés pour les exemples 1 à 9 où ils sont désignés par TAH, TAS et TAF.

Les émulsions obtenues sont observées et les résultats apparaissent au tableau II ci-après.

**TABLEAU II**

| % TA | % Eau | % Fluorohydrocarbure | TAH | TAS | TAF |
|---|---|---|---|---|---|
| 40 | 10 | 50 | (10) -- | (11) ++ | (12) -- |
| 10 | 70 | 20 | (4) - | (5) +++ | (6) -- |
| 5 | 85 | 10 | (13) -- | (14) +++ | (15) -- |

où les nombres indiqués entre parenthèses représentent les numéros des exemples.

### Exemples 16 à 19

Des émulsions sont obtenues de la manière suivante :

On solubilise 3,0 g du tensioactif TAS défini aux exemples précédents dans une solution de 15,0 g du fluorohydrocarbure C défini aux exemples 10 à 15 et 15,0 g de chacune des huiles H₁ à H₄ représentées au tableau III ci-après à une température de 60°C.

On ajoute alors lentement, sous agitation, 67,0 g d'eau en conservant au cours de l'addition une température minimale de 50°C. On laisse alors revenir à une température ambiante en maintenant l'agitation.

Les émulsions obtenues sont observées et les résultats apparaissent au tableau III ci-après.

**TABLEAU III**

| HUILE | EXEMPLE | QUALITE DE L'EMULSION |
|---|---|---|
| H1 Huile de Jojoba | 16 | +++ |
| H2 Isoparaffine hydrogénée | 17 | +++ |
| H3 Cyclopentadiméthylsiloxane | 18 | +++ |
| H4 Perfluorodécaline | 19 | ++ |

### Exemple 20

On solubilise 3,0 g du tensioactif TAS définit ci-dessus dans 30,0 g du fluorohydrocarbure C, à une température de 70°C.

Puis on ajoute lentement sous agitation un mélange de 34,0 g de glycérol avec 33,0 g d'eau en conservant au cours de l'addition une température minimale de 50° C .

On laisse ensuite revenir à température ambiante en maintenant l'agitation.

On obtient alors une émulsion transparente.

### Exemple 21

On solubilise 3,0 g du tensioactif TAS définit ci-dessus dans 30,0 g du fluorohydrocarbure C, à une température de 70°C.

Puis on ajoute lentement sous agitation un mélange de 29,1 g d'hexaglycérol vendu sous la dénomination "Polyglycérine 500" par la Société SAKAMOTO YAKUHIN et 37,9 g d'eau en conservant au cours de l'addition une température minimale de 50°C.

On laisse ensuite revenir à température ambiante en maintenant l'agitation.

On obtient alors une émulsion transparente.

### Exemple 22

On solubilise 10,0 g de lauryldiméthicone copolyol vendu sous la dénomination de "Q2-5200" par la Société DOW CORNING dans 20,0 g du fluorohydrocarbure C à une température de 70°C.

Puis, on ajoute lentement sous agitation 70,0 g d'eau en conservant au cours de l'addition une température minimale de 50°C.

On laisse ensuite revenir progressivement à température ambiante en maintenant l'agitation.

On obtient alors une émulsion fine et stable.

### Exemple 23

On solubilise 10,0 g de decyldimethicone copolyol vendu sous la dénomination de "218-1138" par la Société GENERAL ELECTRIC dans 20,0 g du fluorohydrocarbure C.

Puis, on ajoute lentement sous agitation 70,0 g d'eau en conservant au cours de l'addition une température minimale de 50°C.

On laisse ensuite revenir progressivement à température ambiante en maintenant l'agitation.

On obtient alors une émulsion fine et stable.

### Exemple 24

On solubilise un mélange de 2,5 g de dimethicone copolyol vendu sous la dénomination "KF 60-17" par la Société SHIN-ETSU CHEMICAL et 2,5 g de TAH dans 25,0 g de fluorohydrocarbure C à une température de 70°C.

Puis on ajoute lentement sous agitation 70,0 g d'eau en conservant au cours de l'addition une température minimale de 50°C.

On laisse ensuite revenir progressivement à température ambiante en maintenant l'agitation.

On obtient alors une émulsion fine et stable.

### Exemple 25

On solubilise un mélange de 2,5 g de diméthicone copolyol, vendu sous la dénomination de "SILWET L-722" par la Société UNION CARBIDE, et 2,5 g de TAH dans 25 g de fluorohydrocarbure C à une température de 70°C.

Puis on ajoute lentement sous agitation 70 g d'eau en conservant au cours de l'addition une température minimale de 50°C.

On laisse ensuite revenir à température ambiante en maintenant l'agitation.

On obtient alors une émulsion fine et stable.

### Exemple 26: CREME BLANCHE

### CONSTITUANTS

| Phase H | |
|---|---|
| | % |
| - 1-(2'-F-Hexylethylthio)3-(2''-Ethylhexyloxy)-2 Propanol | 29,40 |
| - Cétyldimethicone copolyol, vendu sous la dénomination d'"Abil EM 90" par la Sté GOLDSCHMIDT | 3,00 |
| - Glycoldistearate + Tristearin, vendu sous la dénomination d'"UNITWIX" par la Société GUARDIAN | 0,50 |
| - Propylparaben. | 0,10 |

| Phase E | |
|---|---|
| - Eau | 61,10 |
| - Sulfate de magnésium | 0,70 |
| - Glycérol | 5,00 |
| - Méthylparaben | 0,20 |
| | 100,00 |

### MODE OPERATOIRE

On pèse ensemble les composés de la phase H que l'on chauffe ensuite à 70°C. Les constituants de la phase E sont aussi pesés ensemble puis chauffés à 80°C.

Après homogénéisation des deux phases, on ajoute lentement la phase E dans la phase H, en agitant à l'aide d'un agiteur du type *Moritz* à une vitesse de 4.000 tr/mn, et en conservant au cours de l'addition, une température minimale de 50°C.

On laisse ensuite revenir progressivement à température ambiante en maintenant l'agitation.

On obtient une crème blanche qui se distingue par de bonnes propriétés d'étalement et de pénétration conduisant à la formation d'un film très doux sur la peau.

### Exemple 27 : CREME BLANCHE

### CONSTITUANTS

| Phase H | |
|---|---|
| | % |
| - 1-(2'-F-Hexyléthylthio)3-(2''-Ethylhexyloxy)-2 Propanol | 29,40 |
| - Laurylméthicone copolyol, vendu sous la dénomination "Q2-5200" par la Société DOW CORNING | 5,00 |
| - Glycoldistéarate + Tristearin, vendu sous la dénomination d'"UNITWIX" par la Société GUARDIAN | 0,50 |
| - Propylparaben | 0,10 |

| Phase E | |
|---|---|
| - Eau | 59,10 |
| - Sulfate de magnésium | 0,70 |
| - Glycérol | 5,00 |
| - Méthylparaben | 0,20 |
| | 100,00 |

Le mode opératoire est le même que celui qui est décrit à l'exemple 26.

### Exemple 28 : CREME BLANCHE

### CONSTITUANTS

| Phase H | |
|---|---|
| | % |
| - 1-(2'-F-Octyléthylthio)3-butyloxy 2-propanol | 29,40 |
| - Cétyldiméthicone copolyol, vendu sous la dénomination d'"ABIL EM 90" par la Société GOLDSCHMIDT | 3,00 |
| - Glycoldistéarate + Tristearin, vendu sous la dénomination d'"UNITWIX" par la Société GUARDIAN | 0,50 |
| - Propylparaben | 0,10 |

| Phase E | |
|---|---|
| - Eau | 61,10 |
| - Sulfate de magnésium | 0,70 |
| - Glycérol | 5,00 |
| - Méthylparaben | 0,20 |
| | 100,00 |

Le mode opératoire est le même que celui décrit dans l'exemple 26.

### Exemple 29 : CREME SOLAIRE

### CONSTITUANTS

| Phase H | |
|---|---|
| | % |
| - 1-(2'-F-Hexyléthylthio)3-(2''-Ethylhexyloxy)-2 Propanol | 12,00 |
| - Cétyldiméthicone copolyol, vendu sous la dénomination "ABIL EM 90" par la Société GOLDSCHMIDT | 3,00 |
| - Glycoldistearate + Tristearin, vendu sous la dénomination d'"UNITWIX" par la Société GUARDIAN | 0,50 |
| - Octylméthoxycinnamate, vendu sous la dénomination de "PARSOL MCX" par la Société GIVAUDAN ROURE | 5,00 |
| - Propylparaben | 0,10 |

| Composé siliconé S | |
|---|---|
| - Cyclopentadiméthylsiloxane | 6,40 |

| Phase E | |
|---|---|
| - Eau | 67,20 |
| - Sulfate de magnésium | 0,70 |
| - Glycérol | 5,00 |
| - Méthylparaben | 0,10 |
| | 100,00 |

### MODE OPERATOIRE

On pèse ensemble les composés de la phase H que l'on chauffe à 70°C.

Après homogénéisation, on la refroidit à 60°C puis on additionne le composé S.

Après homogénéisation, on ajoute lentement la phase E préalablement chauffée à 80°C, puis refroidie à 50°C, en agitant à l'aide d'un agitateur type *Moritz* et en conservant au cours de l'addition à une température minimale de 50°C.

On laisse ensuite revenir progressivement à température ambiante en maintenant l'agitation.

On obtient une crème solaire qui se distingue par de bonnes propriétés d'étalement, conduisant à la formation d'un film très protecteur et très doux sur la peau.

Dans les exemples suivants où interviennent des pigments, ceux-ci sont définis ci-après, avec leur dénomination :

### Enrobage siliconé :

- Oxyde de fer jaune: : "W1802 COVASIL 3.05"
- Oxyde de fer rouge: : "W3801 COVASIL 3.05"
- Oxyde de fer noir: : "W9814 COVASIL 3.05"
- Dioxyde de titane: : "W877 COVASIL 05"

### Enrobage fluoré :

- Oxyde de fer jaune: : "COVAFLUOR"
- Oxyde de fer rouge: : "COVAFLUOR"
- Oxyde de fer noir: : "COVAFLUOR"
- Dioxyde de titane: : "COVAFLUOR"

Les pigments à enrobage sont commercialisés en France par WACKHERR.

### Exemple 30: FOND DE TEINT

### CONSTITUANTS

| Phase H | |
|---|---|
| | % |
| - 1-(2'-F-Hexylethylthio) 3-(2''-Ethylhexyloxy)-2 Propanol | 29,40 |
| - Cetyldimethicone copolyol, vendu sous la dénomination d'"ABIL EM 90" par la Société GOLDSCHMIDT | 3,00 |
| - Glycoldistearate + Tristearin, vendu sous la dénomination d'"UNITWIX" par la Société GUARDIAN | 0,50 |
| - Propylparaben. | 0,10 |

| Phase E | |
|---|---|
| - Eau | 57,20 |
| - Sulfate de magnésium | 0,70 |
| - Glycérol | 5,00 |
| - Méthylparaben | 0,10 |

| Pigments | |
|---|---|
| - Oxyde Fer jaune à enrobage siliconé | 0,60 |
| - oxyde Fer rouge à enrobage siliconé | 0,39 |
| - Oxyde Fer noir à enrobage siliconé | 0,11 |
| - Dioxyde de titane à enrobage siliconé | 2,90 |
| | 100,00 |

### MODE OPERATOIRE

On pèse ensemble les composés de la phase H que l'on chauffe ensuite à 70°C. Les constituants de la phase E sont aussi pesés ensemble puis chauffés à 80°C.

On disperse les pigments dans la phase H en agitant à l'aide d'un agitateur de type *Moritz,* puis, après homogénisation, on ajoute lentement la phase E en maintenant l'agitation et en conservant au cours de l'addition une température minimale de 50°C.

On laisse ensuite revenir progressivement à température ambiante en conservant l'agitation.

On obtient un fond de teint beige rosé qui s'étale bien, qui conduit à un maquillage très naturel et très doux, et qui présente une bonne tenue cosmétique.

### Exemple 31 : FOND DE TEINT

### CONSTITUANTS

| Phase H | |
|---|---|
| | % |
| - 1-(2'-F-Hexyléthylthio)3-(2''-Ethylhexyloxy)-2 Propanol | 29,40 |
| - Cétyldimethicone copolyol, vendu sous la dénomination d'"ABIL EM 90" par la Société GOLDSCHMIDT | 3,00 |
| - Glycoldistearate + Tristearin, vendu sous la dénomination d'"UNITWIX" par la Société GUARDIAN | 0,50 |
| - Propylparaben. | 0,10 |

| Phase E | |
|---|---|
| - Eau | 57,20 |
| - Sulfate de magnésium | 0,70 |
| - Glycérol | 5,00 |
| - Méthylparaben | 0,10 |

| Pigments | |
|---|---|
| - Oxyde Fer jaune à enrobage fluoré | 0,60 |
| - Oxyde Fer rouge à enrobage fluoré | 0,39 |
| - Oxyde Fer noir à enrobage fluoré | 0,11 |
| - Dioxyde de titane à enrobage fluoré | 2,90 |
| | 100,00 |

### MODE OPERATOIRE

Le mode opératoire est identique à celui de l'exemple 30.

On obtient un fond de teint qui a les mêmes caractéristiques que le précédent.

### Exemple 32 : FOND DE TEINT

### CONSTITUANTS

| Phase H | |
|---|---|
| | % |
| - 1-(2'-F-Hexyléthylthio)3-(2''-Ethylhexyloxy)-2 Propanol | 12,00 |
| - Cetyldimethicone copolyol, vendu sous la dénomination d'"ABIL EM 90" par la Société GOLDSCHMIDT | 3,00 |
| - Glycoldistéarate + Tristearin, vendu sous la dénomination d'"UNITWIX" par la Société GUARDIAN | 0,50 |
| - Propylparaben. | 0,10 |

| Composé siliconé S | |
|---|---|
| - Cyclopentadimethylsiloxane | 11,40 |

| Pigments P | |
|---|---|
| - Oxyde Fer jaune à enrobage siliconé | 0,60 |
| - oxyde Fer rouge à enrobage siliconé | 0,39 |
| - Oxyde Fer noir à enrobage siliconé | 0,11 |
| - Dioxyde de titane à enrobage siliconé | 2,90 |

| Phase E | |
|---|---|
| - Eau | 63,20 |
| - Sulfate de magnésium | 0,70 |
| - Glycérol | 5,00 |
| - Méthylparaben | 0,10 |
| | 100,00 |

### MODE OPERATOIRE

On pèse ensemble les composés de la phase H que l'on chauffe à 70°C.

Après homogénéisation, on la refroidit à 60°C puis on additionne le composé siliconé S.

Les pigments P sont ensuite dispersés dans le mélange H + S. Après homogénéisation, on ajoute lentement la phase E, préalablement chauffée à 80°C puis refroidie à 50°C, en agitant à l'aide d'un agitateur type *Moritz* et en conservant au cours de l'addition une température minimale de 50°C.

On laisse ensuite revenir progressivement à température ambiante en conservant l'agitation.

On obtient un fond de teint qui a les mêmes caractéristiques que les précédents.

### Exemple 33 : FOND DE TEINT

### CONSTITUANTS

| Phase H | |
|---|---|
| | % |
| - 1-(2'-F-Hexyléthylthio)3-(2''-Ethylhexyloxy)-2 Propanol | 12,00 |
| - Cétyldiméthicone copolyol, vendu sous la dénomination "ABIL WE09" par la Société GOLDSCHMIDT | 3,00 |
| - Propylparaben | 0,10 |

| Composé siliconé S | |
|---|---|
| - Bentone vendu sous la dénomination "SIMAGEL SI-345" par la Société STEARINERIES DUBOIS | 1,00 |
| - Cyclopentadiméthylsiloxane | 10,90 |

| Pigment P | |
|---|---|
| - Oxyde de fer jaune enrobé de téflon (T-9533 de WARNER-JENKINSON) | 0,60 |
| - Oxyde de fer rouge enrobé de téflon (T-9555 de WARNER-JENKINSON) | 0,39 |
| - Oxyde de fer noir enrobé de téflon (T-9560 de WARNER-JENKINSON) | 0,11 |
| - Dioxyde de titane enrobé de téflon (T-9528 de WARNER-JENKINSON) | 2,90 |

| Phase E | |
|---|---|
| - Eau | 62,20 |
| - Sulfate de magnésium | 0,70 |
| - Glycérol | 5,00 |
| - Méthylparaben | 0,10 |
| | 100,00 |

On opère selon le mode opératoire de l'exemple 30.

## Revendications

1. Emulsion du type E/H, caractérisée en ce qu'elle comporte une phase aqueuse émulsionnée à l'aide d'un tensioactif siliconé, dans une phase continue comportant au moins un fluorohydrocarbure.

2. Emulsion selon la revendication 1, caractérisée en ce que les fluorohydrocarbures ont la formule (I) :
(R_{F})ₓ - (A)_{y} - (R_{H})_{z} (I)
dans laquelle :
x représente 1, 2 ou 3,
y représente 0 ou 1,
z représente 0, 1, 2 ou 3,
à la condition que y et z ne soient pas simultanément 0, et que lorsque z est 0, x est 2 ou 3,
R_{F} représente un radical fluoré aliphatique ou aromatique, saturé ou insaturé, à chaîne linéaire, ramifiée ou cyclique, cette chaîne pouvant être fonctionnalisée et/ou être interrompue par des atomes divalents tels que l'oxygène ou le soufre, ou trivalents tels que l'azote et/ou substituée par des atomes d'hydrogène ou d'autres atomes d'halogène, à la condition que, pour deux atomes de carbone du squelette, ne soit pas présent plus d'un de ces substituants autres que le fluor,
R_{H} représente un radical hydrocarboné aliphatique ou aromatique, saturé ou insaturé, à chaîne linéaire, ramifiée ou cyclique, cette chaîne pouvant être fonctionnalisée et/ou interrompue par un ou plusieurs atomes divalents tels que l'oxygène ou le soufre ou par un ou plusieurs atomes trivalents comme l'azote,
A représente un radical di, tri ou quadrivalent tel que 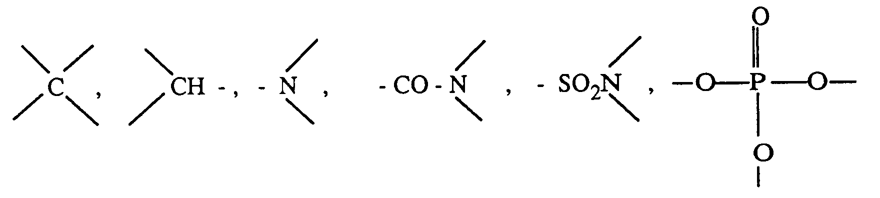 les structures cycliques, aliphatiques ou aromatiques ou les insaturations éthyléniques.

3. Emulsion selon l'une des revendications 1 à 2, caractérisée en ce que le fluorohydrocarbure a pour formule la formule (III) suivante :
R₁ - (CH₂)ₙ-X -[C₃H₅(OH)]-(Y)ₓ - R₂ (III)
où C₃H₅(OH) représente : 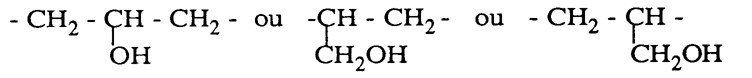
R₁ représente un radical alkyle perfluoré, linéaire ou ramifié en C₄-C₂₀ ou un mélange de radicaux perfluorés, linéaires ou ramifiés en C₄-C₂₀,
R₂ représente un radical alkyle linéaire ou ramifié en C₁-C₂₂ ou un mélange de radicaux alkyle, linéaires ou ramifiés en C₁-C₂₂ ou un radical aryle en C₆-C₁₀ ou aralkyle en C₇-C₁₅,
X et Y, identiques ou différents, représentent : -O-, -S-, 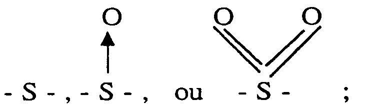 sous réserve que X et Y ne représentent pas simultanément 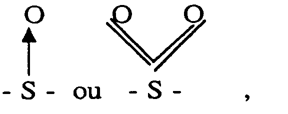
n est compris entre 0 et 4, et
x représente 0 ou 1;
ou la formule (IV) :
R_{F}-(CH₂)ₙ - X - [C₃H₅(OH)]-Y-(CH₂)ₘ-R'_{F} (IV)
dans laquelle C₃H₅(OH) représente les structures : 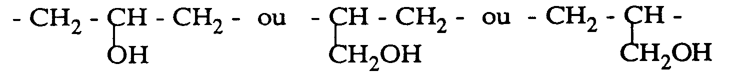
R_{F} et R'_{F}, identiques ou différents, représentent un radical alkyle perfluoré, linéaire ou ramifié en C₄-C₂₀ ou un mélange de radicaux alkyle perfluorés, linéaires ou ramifiés en C₄-C₂₀;
m et n, identiques ou différents, représentent 0, 1, 2, 3 ou 4;
X et Y, identiques, sont - O - ou - S -;
ou la formule (I') :
R_{F}-(CH₂)ₙ -X-[C₃H₅(OH)] -Y-(CH₂)ₘ-R'_{F} (I')
dans laquelle C₃H₅(OH) représente les structures : 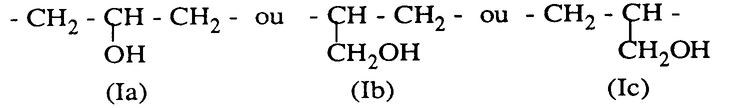
R_{F} et R'_{F}, identiques ou différents, représentent un radical alkyle perfluoré, linéaire ou ramifié en C₄-C₂₀ ou un mélange de radicaux alkyle perfluorés, linéaires ou ramifiés en C₄-C₂₀;
m et n, identiques ou différents, représentent 0, 1, 2, 3 ou 4 et X est O et Y est S ou X est S et Y est O;
ou la formule (V) : 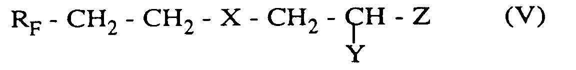
où Y est OH, et
Z est 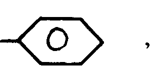 - CH₃, - CH₂OH, - CH₂OCOCH₃
ou bien Y est - CH₂OH et Z est -O-COCH₃
X représente - O - , - S -, 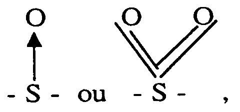 et
R_{F} représente un radical alkyle perfluoré, linéaire ou ramifié, en C₄-C₂₀, ou un mélange de radicaux alkyle perfluorés, linéaires ou ramifiés, en C₄-C₂₀ ; ou la formule (VI) :
R_{F} - CH = CH - CH₂ - O - CH₂ - [C₂H₄ -OW] (VI)
où C₂H₄OW désigne : 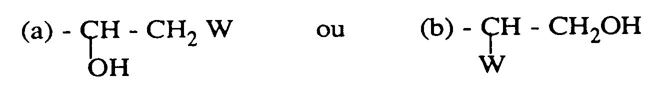
W désignant : -OR, -SR, -COOR, 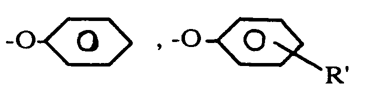
R désigne un radical alkyle en C₁-C₁₈, linéaire ou ramifié,
R' désigne -CH₃ ou -OH, en position ortho ou para, et
R_{F} représente un radical alkyle perfluoré, linéaire ou ramifié, en C₄-C₂₀, ou un mélange de radicaux alkyle perfluorés, linéaires ou ramifiés, en C₄-C₂₀;
ou la formule (VII) : 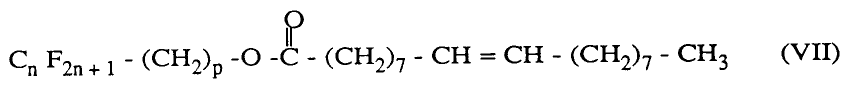 dans laquelle n est un nombre entier égal à 6 ou 8 et p est 1 ou 2.

4. Emulsion selon la revendication 1 ou 3, caractérisée en ce que les fluorohydrocarbures ont un taux de substitution compris entre 0,5 et 95%, de préférence entre 10 et 80%.

5. Emulsion selon l'une des revendications 1 à 4, caractérisée en ce que la phase continue comporte au moins 50 % en poids de fluorohydrocarbure.

6. Emulsion selon l'une des revendications 1 à 5, caractérisée en ce que le tensio-actif siliconé a la formule (II) suivante : 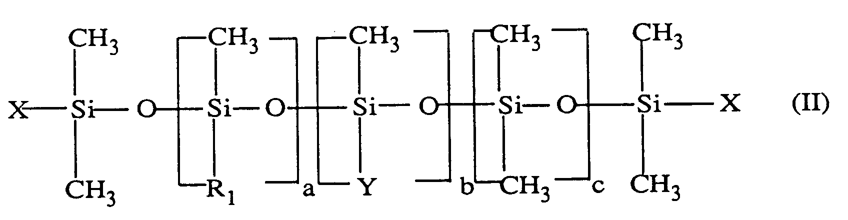 dans laquelle :
X représente CH₃, (CH₂)ₚ-(C₂H₄O)ₘ-(C₃H₆O)ₙ-R₂
Y représente (CH₂)ₚ - O-(C₂H₄O)ₘ-(C₃H₆O)ₙ-R₂
R₁ représente un radical alkyle en C₂-C₁₈
R₂ représente H, un radical alkyle en C₁-C₅, un radical aryle ou un radical acétyle,
p = 1 à 5
m = 0 à 50
n = 0 à 30, m et n n'étant pas simultanément 0,
a = 0 à 100
b = 1 à 50
c = 0 à 300,
ou la formule suivante : 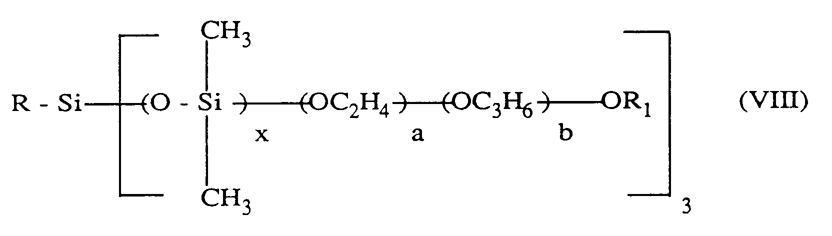 dans laquelle R et R₁ sont des groupes alkyle en C₁ à C₈,
a et b ont une valeur comprise entre 0 et 200 et x a une valeur entre 1 et 100, a et b n'étant pas simultanément nuls.

7. Emulsion selon l'une des revendications 1 à 6, caractérisée en ce que la phase aqueuse représente 10 à 90 % en poids du poids de l'émulsion.

8. Emulsion selon l'une des revendications 1 à 7, caractérisée en ce que les tensioactifs siliconés sont utilisés dans des proportions comprises entre 0, 5 et 40% en poids par rapport au poids de l'émulsion, de préférence entre 2 et 10%.

9. Emulsion selon l'une des revendications 1 à 8, caractérisée en de qu'elle est sous la forme de lait, de crème de soin pour la peau ou les cheveux ou de crème anti-solaire, de crème teintée, de fond de teint, de rouge à lèvres, de mascara ou de fard à joues.

10. Emulsion selon l'une des revendications 1 à 9, caractérisée en ce qu'elle comporte un tensio-actif siliconé de formule (II) où a est nul en association avec au moins un tensio-actif hydrocarboné au HLB inférieur à 7.

11. Emulsion selon l'une des revendications 1 à 10, caractérisée en ce qu'elle comporte en outre au moins un pulvérulent d'origine naturelle ou synthétique.

12. Emulsion selon l'une des revendications 1 à 11, caractérisée en ce qu'elle comporte en outre, dans la phase aqueuse, un excipient choisi parmi les huiles, cires, gélifiants huileux, silicones, filtres, vitamines, hormones, antioxydants, conservateurs, colorants ou parfums et, dans la phase aqueuse, un excipient choisi parmi les polyols, gélifiants, agents gonflants, acides aminés, colorants, et les agents actifs.

13. Utilisation de tensio-actifs siliconés de formule (II) : 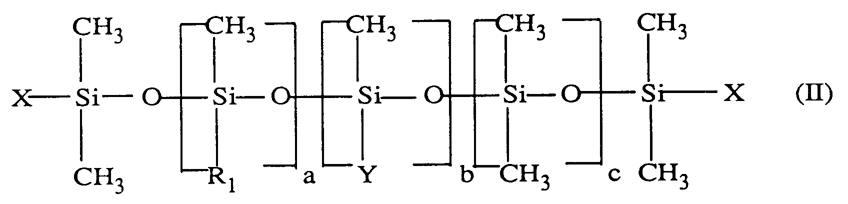 dans laquelle :
X représente CH₃, (CH₂)ₚ-(C₂H₄O)ₘ-(C₃H₆O)ₙ-R₂
Y représente (CH₂)ₚ - O -(C₂H₄O)ₘ-(C₃H₆O)ₙ-R₂
R₁ représente un radical alkyle en C₂-C₁₈
R₂ représente H, un radical alkyle en C₁-C₅, un radical aryle ou un radical acétyle,
p = 1 à 5
m = 0 à 50
n = 0 à 30, m et n n'étant pas simultanément 0
a = 0 à 100
b = 1 à 50
c = 0 à 300
ou de formule (VIII) : 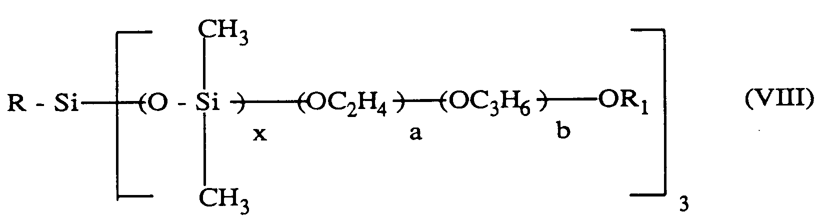 dans laquelle R et R₁ sont des groupes alkyle en C₁ à C₈, a et b ont une valeur comprise entre 0 et 200, a et b n'étant pas simultanément nuls, et x a une valeur comprise entre 1 et 100,
pour la préparation d'émulsion du type eau dans huile comportant une phase aqueuse émulsionnée par ledit tensio-actif de formule (II) ou (VIII), dans une phase grassse continue comportant au moins un fluorohydrocarbure.

## Claims

1. Emulsion of the W/O type, characterized in that it contains an aqueous phase emulsified by means of a silicone surfactant, in a continuous phase containing at least one fluorohydrocarbon.

2. Emulsion according to Claim 1, characterized in that the fluorohydrocarbons are of the formula (I):
(R_{F})ₓ-(A)_{y}-(R_{H})_{z} (I)
in which:
x represents 1, 2 or 3,
y represents 0 or 1,
z represents 0, 1, 2 or 3,
on the condition that y and z are not simultaneously 0, and that when z is 0, x is 2 or 3,
R_{F} represents a saturated or unsaturated, aliphatic or aromatic fluorinated radical with a linear, branched or cyclic chain, it being possible for this chain to be functionalized and/or to be interrupted by divalent atoms such as oxygen or sulphur, or trivalent atoms such as nitrogen and/or substituted by hydrogen atoms or other halogen atoms, on the condition that, for two carbon atoms of the skeleton, not more than one of these substituents other than fluorine is present,
R_{H} represents a saturated or unsaturated, aliphatic or aromatic hydrocarbon radical with a linear, branched or cyclic chain, it being possible for this chain to be functionalized and/or interrupted by one or more divalent atoms such as oxygen or sulphur or by one or more trivalent atoms such as nitrogen,
A represents a di-, tri- or quadrivalent radical such as 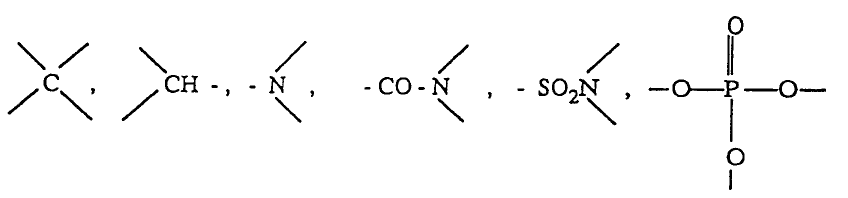 cyclic, aliphatic or aromatic structures or ethylenic unsaturations.

3. Emulsion according to one of Claims 1 to 2, characterized in that the formula for the fluorohydrocarbon is the following formula (III):
R₁ - (CH₂)ₙ - X -[C₃H₅(OH)]- (Y)ₓ - R₂ (III)
where C₃H₅(OH) represents: 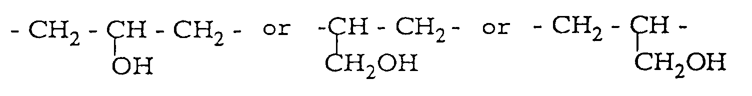
R₁ represents a linear or branched perfluorinated C₄-C₂₀ alkyl radical or a mixture of linear or branched perfluorinated C₄-C₂₀ radicals,
R₂ represents a linear or branched C₁-C₂₂ alkyl radical or a mixture of linear or branched C₁-C₂₂ alkyl radicals or a C₆-C₁₀ aryl or C₇-C₁₅ aralkyl radical,
X and Y, which are identical or different, represent: - O - , - S - 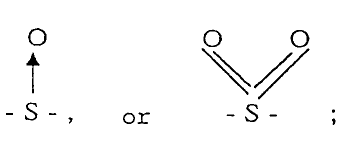 , provided that X and Y do not simultaneously represent 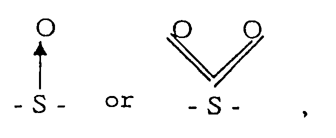
n is between 0 and 4, and
x represents 0 or 1; or the formula (IV):
R_{F} - (CH₂)ₙ - X - [C₃H₅(OH)] - Y - (CH₂)ₘ - R'_{F} (IV)
in which C₃H₅(OH) represents the structures: 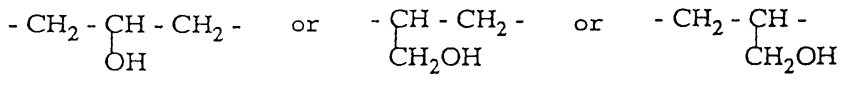
R_{F} and R'_{F}, which are identical or different, represent a linear or branched perfluorinated C₄-C₂₀ alkyl radical or a mixture of linear or branched perfluorinated C₄-C₂₀ alkyl radicals;
m and n, which are identical or different, represent 0, 1, 2, 3 or 4;
X and Y, which are identical, are -O- or -S-; or the formula (I')
R_{F} - (CH₂)ₙ - X - [C₃H₅(OH)] - Y - (CH₂)ₘ - R'_{F} (I')
in which C₃H₅(OH) represents the structures: 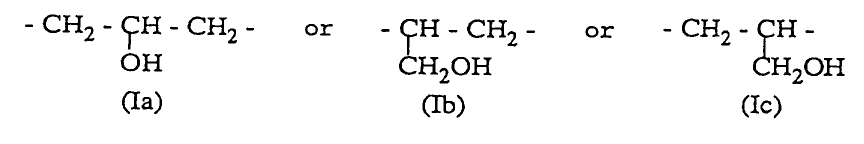
R_{F} and R'_{F}, which are identical or different, represent a linear or branched perfluorinated C₄-C₂₀ alkyl radical or a mixture of linear or branched perfluorinated C₄-C₂₀ alkyl radicals;
m and n, which are identical or different, represent 0, 1, 2, 3 or 4 and X is O and Y is S or X is S and Y is O;
or the formula (V): 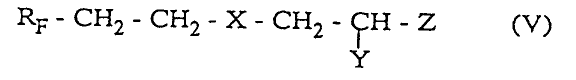 where Y is OH, and
Z is 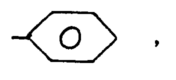 - CH₃, -CH₂OH, - CH₂OCOCH₃
or alternatively Y is -CH₂OH and Z is -O-COCH₃
X represents - O - , - S - 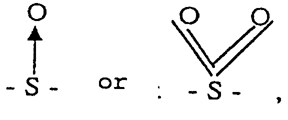 , and
R_{F} represents a linear or branched perfluorinated C₄-C₂₀ alkyl radical, or a mixture of linear or branched perfluorinated C₄-C₂₀ alkyl radicals;
or the formula (VI):
R_{F} - CH = CH - CH₂ - O - CH₂ - [C₂H₄ - OW] (VI)
where
C₂H₄OW designates: 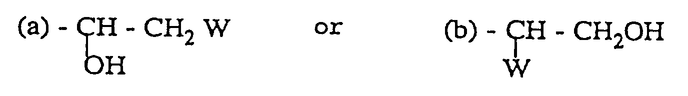
W designating: -OR, -SR, -COOR, 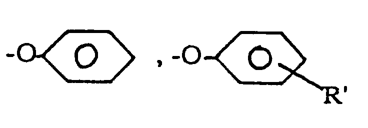
R designates a linear or branched C₁-C₁₈ alkyl radical,
R' designates -CH₃ or -OH, in the ortho or para position, and
R_{F} represents a linear or branched perfluorinated C₄-C₂₀ alkyl radical, or a mixture of linear or branched perfluorinated C₄-C₂₀ alkyl radicals;
or the formula (VII): 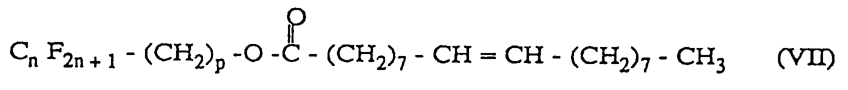 in which n is an integer equal to 6 or 8 and p is 1 or 2.

4. Emulsion according to Claim 1 or 3, characterized in that the fluorohydrocarbons have a substitution rate of between 0.5 and 95%, preferably between 10 and 80%.

5. Emulsion according to one of Claims 1 to 4, characterized in that the continuous phase contains at least 50% by weight of fluorohydrocarbon.

6. Emulsion according to one of Claims 1 to 5, characterized in that the silicone surfactant is of the following formula (II): 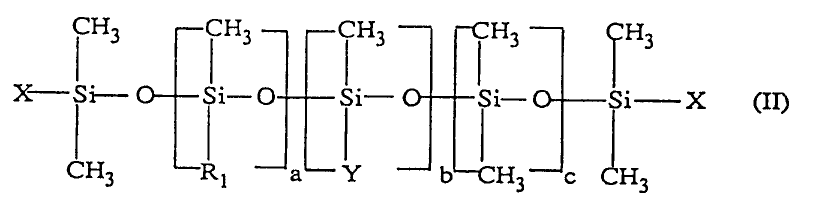 in which
X represents CH₃, (CH₂)ₚ-(C₂H₄O)ₘ-(C₃H₆O)ₙ-R₂
Y represents (CH₂)ₚ-O-(C₂H₄O)ₘ-(C₃H₆O)ₙ-R₂
R₁ represents a C₂-C₁₈ alkyl radical
R₂ represents H, a C₁-C₅ alkyl radical, an aryl radical or an acetyl radical,
p = 1 to 5
m = 0 to 50
n = 0 to 30, m and n not simultaneously being 0
a = 0 to 100
b = 1 to 50
c = 0 to 300,
or the following formula: 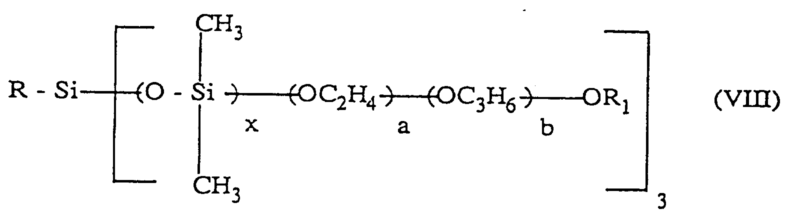 in which R and R₁ are C₁ to C₈ alkyl groups,
a and b have a value between 0 and 200 and x has a value between 1 and 100, a and b not simultaneously being zero.

7. Emulsion according to one of Claims 1 to 6, characterized in that the aqueous phase represents 10 to 90% by weight of the weight of the emulsion.

8. Emulsion according to one of Claims 1 to 7, characterized in that the silicone surfactants are used in proportions of between 0.5 and 40% by weight relative to the weight of the emulsion, preferably between 2 and 10%.

9. Emulsion according to one of Claims 1 to 8, characterized in that it is in the form of a milk, a cream for skin or hair care, anti-sun cream, coloured cream, foundation, lipstick, mascara or blusher.

10. Emulsion according to one of Claims 1 to 9, characterized in that it contains a silicone surfactant of formula (II) where a is zero in combination with at least one hydrocarbon surfactant with an HLB less than 7.

11. Emulsion according to one of Claims 1 to 10, characterized in that it contains, in addition, at least one pulverulent product of natural or synthetic origin.

12. Emulsion according to one of Claims 1 to 11, characterized in that it contains, in addition, in the aqueous phase, an excipient chosen from oils, waxes, oily gelling agents, silicones, screening agents, vitamins, hormones, antioxidants, preservatives, colorants or perfumes and, in the aqueous phase, an excipient chosen from polyols, gelling agents, swelling agents, amino acids, colorants and active agents.

13. Use of silicone surfactants of formula (II): 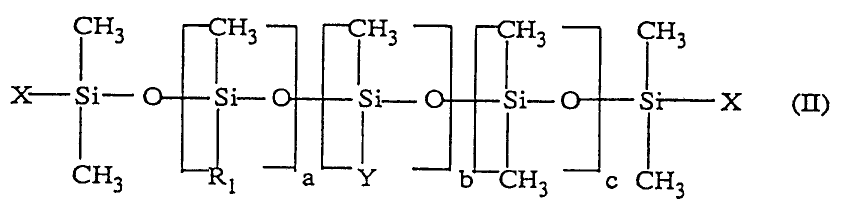 in which: H₆O)ₙ-R₂
X represents CH₃, (CH₂)ₚ-(C₂H₄O)ₘ-(C₃H₆O)ₙ-R₂
Y represents (CH₂)ₚ-O-(C₂H₄O)ₘ-(C₃H₆O)ₙ-R₂
R₁ represents a C₂-C₁₈ alkyl radical
R₂ represents H, a C₁-C₅ alkyl radical, an aryl radical or an acetyl radical,
p = 1 to 5
m = 0 to 50
n = 0 to 30, m and n not simultaneously being 0
a = 0 to 100
b = 1 to 50
c = 0 to 300
or of formula (VIII): 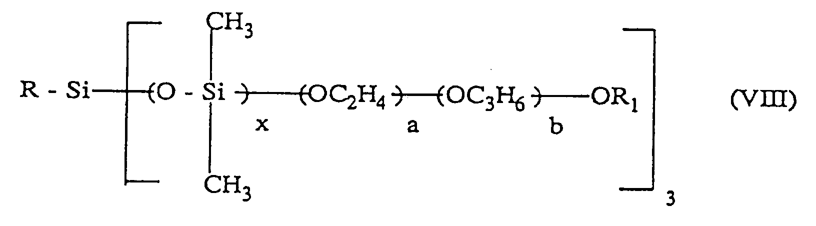 where R and R₁ are C₁ to C₈ alkyl groups, a and b have a value between 0 and 200, a and b not simultaneously being zero, and x has a value between 1 and 100,
for the preparation of a water-in-oil type emulsion containing an aqueous phase emulsified by the said surfactant of formula (II) or (VIII), in a continuous fatty phase containing at least one fluorohydrocarbon.

## Patentansprüche

1. Emulsion des W/O-Typs, dadurch gekennzeichnet, daß sie eine wäßrige Phase enthält, welche mit Hilfe eines Silicon-Tensids in einer kontinuierlichen Phase, die mindestens einen Fluorkohlenwasserstoff enthält, emulgiert ist.

2. Emulsion nach Anspruch 1, dadurch gekennzeichnet, daß die Fluorkohlenwasserstoffe die Formel (I) haben:
(R_{F})ₓ-(A)_{y}-(R_{H})_{z} (I)
in der
x 1,2 oder 3 darstellt;
y 0 oder 1 darstellt;
z 0, 1, 2 oder 3 darstellt, unter der Bedingung, daß y und z nicht gleichzeitig 0 sind, und daß, wenn z 0 ist,
x 2 oder 3 ist;
R_{F} ein aliphatisches oder aromatisches, gesättigtes oder ungesättigtes, lineares, verzweigtes oder cyclisches fluoriertes Radikal darstellt, wobei diese Kette durch zweiwertige Atome, wie z.B. Sauerstoff oder Schwefel, oder durch dreiwertige Atome, wie z.B. Stickstoff, funktionalisiert und/oder unterbrochen sein kann und/oder durch Wasserstoffatome oder andere Halogenatome substituiert sein kann, unter der Bedingung, daß für zwei Kohlenstoffatome des Kohlenstoffgerüsts nicht mehr als ein Substituent ein anderer als Fluor ist;
R_{H} ein aliphatisches oder aromatisches, gesättigtes oder ungesättigtes, lineares, verzweigtes oder cyclisches Kohlenwasserstoffradikal darstellt, wobei diese Kette durch ein oder mehrere zweiwertige Atome, wie z.B. Sauerstoff oder Schwefel, oder durch ein oder mehrere dreiwertige Atome, wie Stickstoff, funktionalisiert und/oder unterbrochen ist;
A ein zweiwertiges, dreiwertiges oder vierwertiges Radikal darstellt, z.B.: 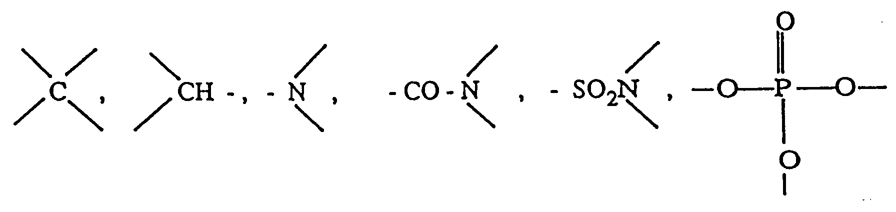 wobei die Strukturen cyclisch, aliphatisch oder aromatisch sind, und die ungesättigten Strukturen ethylenartig sind.

3. Emulsion nach Anspruch 1 oder 2, dadurch gekennzeichnet, daß der Fluorkohlenwasserstoff die folgende Formel (III):
R₁-(CH₂)ₙ-X-[C₃H₅(OH)]-(Y)ₓ-R₂ (III)
in der C₃H₅(OH) für 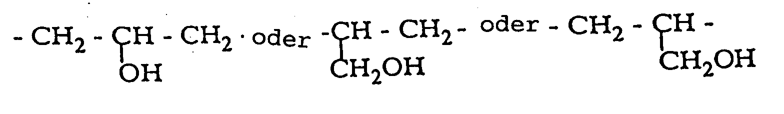 steht;
R₁ ein perfluoriertes, lineares oder verzweigtes C₄-C₂₀-Alkylradikal oder ein Gemisch aus perfluorierten, linearen oder verzweigten C₄-C₂₀-Radikalen darstellt;
R₂ ein geradkettiges oder verzweigtes C₁-C₂₂-Alkylradikal oder ein Gemisch aus linearen oder verzweigten C₁-C₂₂-Alkylradikalen oder ein C₆-C₁₀-Arylradikal oder ein C₇-C₁₅-Aralkylradikal darstellt;
X und Y, die gleich oder verschieden sind, -O-, -S-, 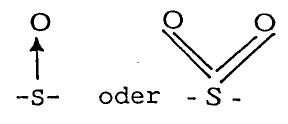 darstellen, unter den Vorbehalt, daß X und Y nicht gleichzeitig 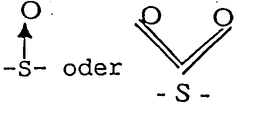 darstellen;
n zwischen 0 und 4 liegt, und
x 0 oder 1 darstellt;
oder die Formel (IV):
R_{F}-(CH₂)ₙ-X-[C₃H₅(OH)]-Y-(CH₂)ₘ-R'_{F} (IV)
in der C₃H₅(OH) die folgenden Strukturen darstellt: 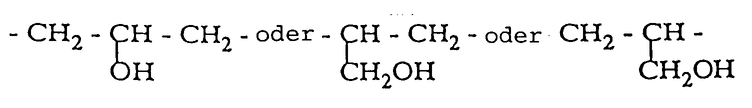
R_{F} und R'_{F}, die gleich oder verschieden sind, ein perfluoriertes, lineares oder verzweigtes C₄-C₂₀-Alkylradikal oder ein Gemisch aus perfluorierten, linearen oder verzweigten C₄-C₂₀-Alkylradikalen darstellen;
m und n, die gleich oder verschieden sind, 0, 1, 2, 3 oder 4 darstellen;
X und Y, die gleich sind, -O- oder -S- sind;
oder die Formel (I'):
R_{F}-(CH₂)ₙ-X-[C₃H₅(OH)]-Y-(CH₂)ₘ-R'_{F} (I')
in der C₃H₅(OH) die folgenden Strukturen darstellt: 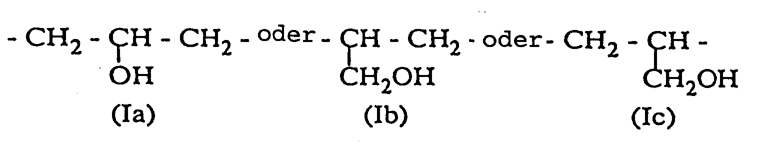
R_{F} und R'_{F}, die gleich oder verschieden sind, ein perfluoriertes, lineares oder verzweigtes C₄-C₂₀-Alkylradikal oder ein Gemisch aus perfluorierten, linearen oder verzweigten C₄-C₂₀-Alkylradikalen darstellen;
m und n, die gleich oder verschieden sind, 0, 1, 2, 3 oder 4 darstellen, und X O ist und Y S ist, oder X S ist, und Y O ist,
oder die Formel (V): 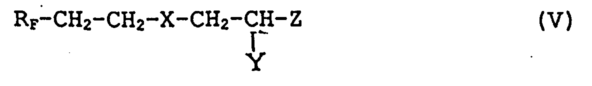 in der Y OH ist, und 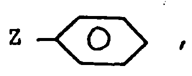 -CH₃, -CH₂OH, -CH₂OCOCH₃ ist;
oder Y auch -CH₂OH ist, und Z -O-COCH₃ ist;
X -O-, -S-, 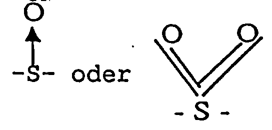 darstellt, und
R_{F} ein perfluoriertes, lineares oder verzweigtes C₄-C₂₀-Alkylradikal oder ein Gemisch aus perfluorierten, linearen oder verzweigten C₄-C₂₀-Alkylradikalen darstellt;
oder die Formel (VI):
R_{F}-CH=CH-CH₂-O-CH₂-[C₂H₄-OW] (VI)
in der
C₂H₄OW: 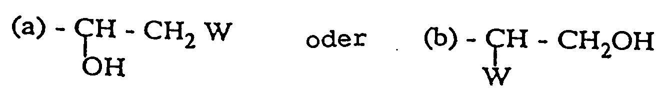 bezeichnet, worin W:
-OR, -SR, -COOR, 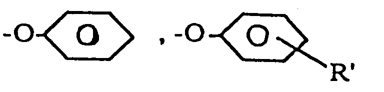 in welchen R ein lineares oder verzweigtes C₁-C₈-Alkylradikal darstellt, R' -CH₃ oder -OH in ortho- oder para-Stellung bezeichnet, darstellt; und
R_{F} ein perfluoriertes, lineares oder verzweigtes C₄-C₂₀-Alkylradikal oder ein Gemisch aus perfluorierten, linearen oder verzweigten C₄-C₂₀-Alkylradikalen darstellt;
oder die Formel (VII) hat: 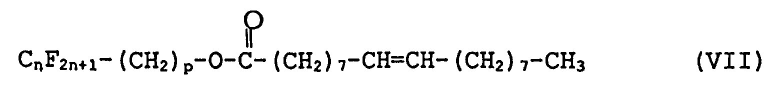 in der n die ganze Zahl 6 oder 8 ist, und p 1 oder 2 ist.

4. Emulsion nach Anspruch 1 oder 3, dadurch gekennzeichnet, daß die Fluorkohlenwasserstoffe einen Substitutionsgrad haben, der zwischen 0,5 und 95%, vorzugsweise zwischen 10 und 80%, liegt.

5. Emulsion nach einem der Ansprüche 1 bis 4, dadurch gekennzeichnet, daß die kontinuierliche Phase mindestens 50 Gew.-% Fluorkohlenwasserstoffe enthält.

6. Emulsion nach einem der Ansprüche 1 bis 5, dadurch gekennzeichnet, daß das Silicon-Tensid die folgende Formel (II) hat: 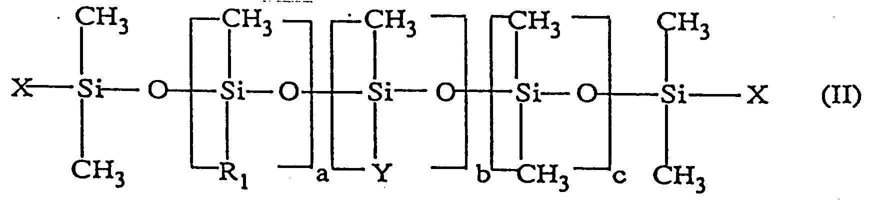 in der
X CH₃, (CH₂)ₚ-(C₂H₄O)ₘ-(C₃H₆O)ₙ-R₂ darstellt,
Y (CH₂)ₚ-O-(C₂H₄O)ₘ-(C₃H₆O)ₙ-R₂ darstellt,
R₁ ein C₂-C₁₈-Alkylradikal darstellt,
R₂ H, ein C₁-C₅-Alkylradikal, ein Arylradikal oder ein Acetylradikal darstellt,
p = 1 bis 5,
m = 0 bis 50,
n = 0 bis 30, wobei m und n nicht gleichzeitig 0 sind,
a = 0 bis 100,
b = 1 bis 50,
c = 0 bis 300,
oder die folgende Formel (VIII) hat: 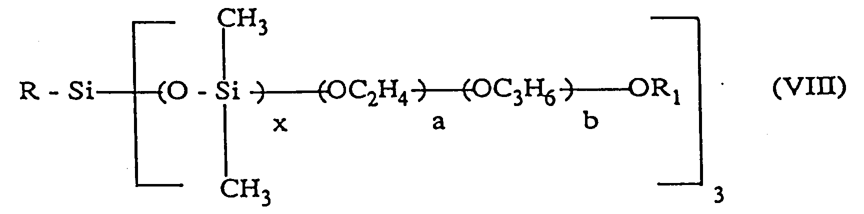 in der R und R₁ C₁-C₈-Alkylgruppen sind, a und b einen Wert haben, der zwischen 0 und 200 liegt, und x einen Wert hat, der zwischen 1 und 100 liegt, und a und b nicht gleichzeitig null sind.

7. Emulsion nach einem der Ansprüche 1 bis 6, dadurch gekennzeichnet, daß die wäßrige Phase 10 bis 90 Gew.-% des Gewichtes der Emulsion ausmacht.

8. Emulsion nach einem der Ansprüche 1 bis 7, dadurch gekennzeichnet, daß die Silicon-Tenside in Verhältnissen zwischen 0,5 und 40 Gew.-%, vorzugsweise 2 und 10%, bezogen auf das Gewicht der Emulsion, verwendet werden.

9. Emulsion nach einem der Ansprüche 1 bis 8, dadurch gekennzeichnet, daß sie als Milch, Pflegecreme für die Haut oder die Haare oder als Sonnenschutzcreme, getönte Creme, Make-up-Grundierung, Lippenstift, Wimperntusche oder Spielschminke vorliegt.

10. Emulsion nach einem der Ansprüche 1 bis 9, dadurch gekennzeichnet, daß sie ein Silicon-Tensid der Formel (II), in der a null ist, in Kombination mit mindestens einem Kohlenwasserstoff-Tensid, das einen HLB-Wert unter 7 hat, enthält.

11. Emulsion nach einem der Ansprüche 1 bis 10, dadurch gekennzeichnet, daß sie zusätzlich mindestens ein Pulver natürlicher oder synthetischer Herkunft enthält.

12. Emulsion nach einem der Ansprüche 1 bis 11, dadurch gekennzeichnet, daß sie in der wäßrigen Phase außerdem einen Zusatzstoff, der aus Ölen, Wachsen, ölhaltigen Geliermitteln, Siliconen, Filtersubstanzen, Vitaminen, Hormonen, Antioxidanzien, Konservierungsstoffen, Farbstoffen und Parfums ausgewählt ist, und in der wäßrigen Phase einen Zusatzstoff, der aus den Polyolen, Geliermitteln, Treibmitteln, Aminosäuren, Farbstoffen und Wirkstoffen ausgewählt ist, enthält.

13. Verwendung von Silicon-Tensiden der Formel (II): 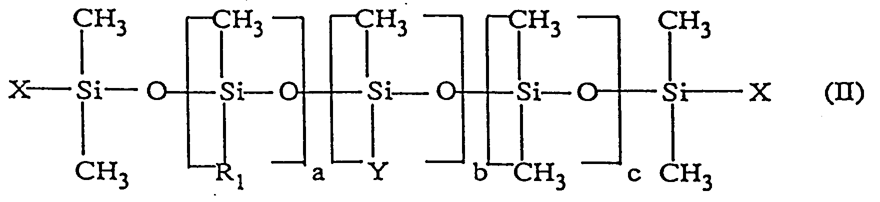 in der
X CH₃, (CH₂)ₚ-(C₂H₄O)ₘ-(C₃H₆O)ₙ-R₂ darstellt,
Y (CH₂)ₚ-O-(C₂H₄O)ₘ-(C₃H₆O)ₙ-R₂ darstellt,
R₁ ein C₂-C₁₈-Alkylradikal darstellt,
R₂ H, ein C₁-C₅-Alkylradikal, ein Arylradikal oder ein Acetylradikal darstellt,
p = 1 bis 5,
m = 0 bis 50,
n = 0 bis 30, wobei m und n nicht gleichzeitig null sind,
a = 0 bis 100,
b = 1 bis 50,
c = 0 bis 300,
oder der Formel (VIII): 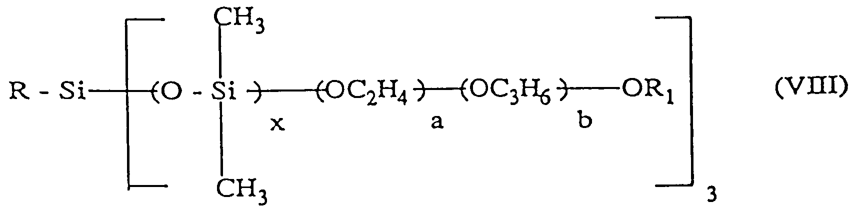 in der R und R₁ C₁-C₈-Alkylgruppen sind, a und b einen Wert zwischen 0 und 200 haben, a und b nicht gleichzeitig null sind, und x einen Wert zwischen 1 und 100 hat,
zur Herstellung einer Emulsion des Wasser-in-Öl-Typs, die eine wäßrige Phase enthält, die durch das genannte Tensid der Formel (II) oder (VIII) in einer kontinuierlichen fetten Phase, die mindestens einen Fluorkohlenwasserstoff enthält, emulgiert ist.
